# FASCICULE DE BREVET EUROPEEN

(11) **EP 0 931 541 B1**
(45) Date de publication et mention de la délivrance du brevet: **16.04.2003**
(21) Numéro de dépôt: 98402902.5
(22) Date de dépôt: 20.11.1998
(51) Int. Cl.: A61K 7/48, A61K 7/06

(54) **Composition contenant une association de procystéine et de polyol**
Mittel enthaltend eine Kombination aus Procystein und einem Polyol
Composition containing a procysteine and polyol combination

(30) Priorité: 05.01.1998 FR 9800019
(43) Date de publication de la demande: 28.07.1999
(73) Titulaire: L'OREAL, 75008 Paris (FR)
(72) Inventeur: Chevalier, Véronique, 94440 Villecresnes (FR); Galey, Jean-Baptiste, 93600 Aulnay Sous Bois (FR)
(74) Mandataire: Renard, Emmanuelle

(56) Documents cités:
- EP-A- 0 656 201
- EP-A- 0 780 120
- US-E- R E34 185

## Description

Il est connu d'utiliser l'acide L-2-oxothiazolidine 4-carboxylique dans des compositions cosmétiques ou dermatologiques destinées à une application topique.

De telles compositions peuvent être destinées à prévenir la chute des cheveux ou à stimuler la repousse des cheveux, comme il est décrit dans EP-656 201.

La procystéine est également connue par le brevet FR-2 742 658 comme agent dépigmentant ou blanchissant de la peau humaine.

Cependant, la procystéine présente une certaine instabilité, en particulier lorsqu'elle est en présence d'eau. Aussi, lorsqu'elle est introduite dans une composition cosmétique, en particulier une composition comprenant de l'eau, son efficacité diminue au cours du temps. En outre, la composition dans laquelle elle est introduite, après un certain temps de stockage, présente des signes de dégradation : coloration, odeur, qui sont inacceptables pour l'utilisateur.

Par conséquent, à l'heure actuelle, il subsiste le besoin d'une composition utilisable dans les domaines cosmétique et dermatologique, contenant de l'acide L-2-oxothiazolidine 4-carboxylique, ce dernier restant stable au cours du temps.

La demanderesse a maintenant découvert que l'association de l'acide L-2-oxothiazolidine 4-carboxylique (I) avec un polyol (II), dans des quantités en poids particulières, présentait une stabilité inattendue, en particulier lorsque cette association est introduite dans un milieu comportant de l'eau.

L'invention a donc pour premier objet l'association de l'acide L-2-oxothiazolidine 4-carboxylique ou procystéine, désigné composé (I) avec au moins un composé appartenant à la classe des polyols, désigné composé (II), les quantités en poids des composés (I) et (II) étant désignées respectivement [I] et [II] et vérifiant la relation [I]/[II] ≤ 1/4.

De préférence [I]/[II] ≤ 1/5, et encore plus préférentiellement [I]/[II] ≤ 1/10.

L'invention a également pour objet une composition cosmétique comprenant l'association ci-dessus et de l'eau. Cette composition comprend un support cosmétiquement acceptable, dont l'eau est un des constituants. Les éléments du support seront choisis en particulier de telle sorte que les compositions selon l'invention soient non irritantes, non toxiques, non allergisantes.

Préférentiellement, la composition selon l'invention comprend :
- de l'acide L-2-oxothiazolidine 4-carboxylique (I)
- au moins un polyol (II)
- de l'eau
les quantités en poids des composants (I) et (II) étant désignées respectivement [I] et [II] et vérifiant : [I]/[II] ≤ 1/4,
la quantité d'eau étant inférieure ou égale à 35 % en poids par rapport au poids total de la composition, de préférence inférieure ou égale à 30 %, encore plus préférentiellement inférieure ou égale à 20%.

De telles compositions permettent de véhiculer la procystéine de façon stable au cours du temps sans perte d'efficacité de cet actif.

Par polyol, on désigne un composé de type alkyle, linéaire ramifié ou cyclique, saturé ou insaturé portant au moins deux fonctions -OH sur la chaine alkyle, ainsi que les polymères (polyéthers) de ces composés alkyles polyhydroxylés. De préférence il s'agit d'un composé alkyle ayant de 2 à 12 atomes de carbone, et encore plus préférentiellement de 2 à 8 atomes de carbone. Avantageusement, ce composé alkyle comporte 2 ou 3 atomes de carbone.

Le polyol utilisé selon l'invention peut être choisi notamment parmi : le glycérol, l'éthylène glycol, le propylène glycol, les polymères et les copolymères du glycérol, de l'éthylène glycol et du propylène glycol, comme par exemple le dipropylène glycol et l'hexaglycérol.

On peut citer également parmi les polyols utilisables selon l'invention : le sorbitol, l'hexylène glycol, le butylène glycol, le pentylène glycol, le butyldiglycol, le 1,2,3-trihydroxyhexane.

L'invention a plus particulièrement pour objet une composition telle que décrite ci-dessus caractérisée en ce qu'elle est destinée à la dépigmentation ou le blanchiment de la peau humaine ou des poils et des cheveux humains.

L'invention a également pour objet une composition telle que décrite ci-dessus, caractérisée en ce qu'elle est destinée à prévenir la chute des cheveux ou à stimuler la repousse des cheveux.

Avantageusement, la composition selon l'invention comprend de 0,01 à 10 % en poids d'acide L-2-oxothiazolidine 4-carboxylique, préférentiellement de 0,1 à 5 %, encore plus préférentiellement de 0,5 à 3 % en poids par rapport au poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou d'une émulsion multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. On peut également associer l'association selon l'invention à d'autres agents dépigmentants, tels que l'acide kojique ou l'hydroquinone et ses dérivés, ce qui permet d'utiliser ces derniers à des doses moins toxiques pour la peau. On peut aussi associer l'association selon l'invention à d'autres agents anti-chute et/ou repousse des cheveux. En cas d'incompatibilité, ces actifs et/ou l'association selon l'invention peuvent être incorporés dans des sphérules, notamment des vésicules ioniques ou non-ioniques et/ou des nanoparticules (nanocapsules et/ou nanosphères), de manière à les isoler les uns des autres dans la composition.

Avantageusement, la phase aqueuse de la composition est neutralisée par ajustement de son pH à une valeur comprise entre 6 et 8.

L'invention a également pour objet l'utilisation d'au moins un polyol pour stabiliser la procystéine.

L'invention a en outre pour objet un procédé de stabilisation de la procystéine dans une composition cosmétique, ce procédé consistant à incorporer à ladite composition au moins un composé choisi parmi les polyols.

### EXEMPLES

Les exemples suivants sont donnés à titre d'illustration de l'invention. Les pourcentages sont donnés en poids par rapport au poids total de chaque constituant de la composition.

### EXEMPLE 1: Crème dépigmentante visage :

| | |
|---|---|
| Procystéine | 1 % |
| Huile d'abricot | 3 % |
| Triéthanolamine | 1 % |
| Aluminium starch octényl succinate | 3 % |
| Poudre de Nylon | 7 % |
| Cyclométhicone et diméthicone copolyol | 20 % |
| Phényl triméthicone | 4 % |
| Propylène glycol | 6 % |
| Glycérine | 23 % |
| EDTA | q.s. |
| Conservateurs | q.s. |
| Eau | 31,5 % |

### EXEMPLE 2 : Crème dépigmentante visage :

| | |
|---|---|
| Procystéine | 1 % |
| Huile d'abricot | 3 % |
| Charges | 10 % |
| Cyclométhicone | 12,5 % |
| Cyclométhicone et diméthicone copolyol | 20 % |
| Phényl triméthicone | 4 % |
| Propylène glycol | 6 % |
| Glycérine | 23 % |
| EDTA | q.s. |
| Conservateurs | q.s. |
| Eau | 19% |

## Revendications

1. Association **caractérisée par le fait qu'**elle consiste en au moins de l'acide L-2-oxothiazolidine 4-carboxylique désigné composé (I) avec au moins un composé appartenant à la classe des polyols, désigné composé (II), les quantités en poids des composés (I) et (II) étant désignées respectivement [I] et [II] et vérifiant la relation [I]/[II] ≤ 1/4.

2. Association selon la revendication 1, **caractérisée en ce que** [I]/[II] ≤ 1/5, préférentiellement [I]/[II] ≤ 1/10.

3. Association selon l'une quelconque des revendications précédentes, **caractérisée par le fait que** le polyol est un composé de type alkyle, linéaire ramifié ou cyclique, saturé ou insaturé ayant de 2 à 12 atomes de carbone, portant au moins deux fonctions -OH sur la chaine alkyle, ainsi que les polymères de ces composés alkyles polyhydroxylés.

4. Association selon la revendication précédente, **caractérisée par le fait que** le polyol est choisi parmi : le glycérol, l'éthylène glycol, le propylène glycol, les polymères et les copolymères du glycérol, de l'éthylène glycol et du propylène glycol, le sorbitol, l'hexylène glycol, le butylène glycol, le pentylène glycol, le butyldiglycol, le 1,2,3-trihydroxyhexane.

5. Composition cosmétique **caractérisée par le fait qu'**elle comprend :
- de l'acide L-2-oxothiazolidine 4-carboxylique (I)
- au moins un polyol (II)
- de l'eau,
les quantités en poids des composants (I) et (II) étant désignées respectivement [I] et [II] et vérifiant : [I]/[II] ≤ 1/4.

6. Composition cosmétique selon la revendication 5, **caractérisée par le fait que** la quantité d'eau est inférieure ou égale à 35 % en poids par rapport au poids total de la composition, de préférence inférieure ou égale à 30 %, encore plus préférentiellement inférieure ou égale à 20%.

7. Composition selon l'une quelconque des revendications 5 et 6, **caractérisée en ce qu'**elle est destinée à la dépigmentation ou le blanchiment de la peau humaine ou des poils et des cheveux humains.

8. Composition selon l'une quelconque des revendications 5 et 6, **caractérisée en ce qu'**elle est destinée à prévenir la chute des cheveux ou à stimuler la repousse des cheveux.

9. Composition selon l'une quelconque des revendications 5 à 8, **caractérisée en ce qu'**elle comprend de 0,01 à 10 % en poids d'acide L-2-oxothiazolidine 4-carboxylique, préférentiellement de 0,1 à 5 %, encore plus préférentiellement de 0,5 à 3 % en poids par rapport au poids total de la composition.

10. Composition selon l'une quelconque des revendications 5 à 9, **caractérisée en ce que** la phase aqueuse est neutralisée par ajustement de son pH à une valeur comprise entre 6 et 8.

11. Utilisation d'au moins un polyol pour stabiliser la procystéine.

12. Procédé de stabilisation de la procystéine dans une composition cosmétique, ce procédé consistant à incorporer à ladite composition au moins un composé choisi parmi les polyols.

## Patentansprüche

1. Kombination, **dadurch gekennzeichnet, dass** sie zumindest aus L-2-Oxothiazolidin-4-carbonsäure, die als Verbindung (I) bezeichnet wird, und aus mindestens einer Verbindung, die zur Gruppe der Polyole gehört und als Verbindung (II) bezeichnet wird, besteht, wobei die Gewichtsanteile der Verbindungen (I) und (II) als [I] bzw. [II] bezeichnet werden und die Beziehung [I]/[II] ≤ 1/4 erfüllen.

2. Kombination nach Anspruch 1, **dadurch gekennzeichnet, dass** [I]/(II] ≤ 1/5 und vorzugsweise [I]/[II] ≤ 1/10 ist.

3. Kombination nach einem der vorhergehenden Ansprüche, **dadurch gekennzeichnet, dass** es sich bei dem Polyol um eine geradkettige, verzweigte oder cyclische, gesättigte oder ungesättigte Verbindung vom Alkyltyp mit 2 bis 12 Kohlenstoffatomen, die an der Alkylkette mindestens zwei -OH-Gruppen trägt, sowie um Polymere dieser Alkylverbindungen, die polyhydroxyliert sind, handelt.

4. Kombination nach dem vorhergehenden Anspruch, **dadurch gekennzeichnet, dass** das Polyol unter Glycerin, Ethylenglykol, Propylenglykol, Polymeren und Copolymeren von Glycerin, Ethylenglykol und Propylenglykol, Sorbit, Hexylenglykol, Butylenglykol, Pentylenglykol, Butyldiglykol und 1,2,3-Trihydroxyhexan ausgewählt ist.

5. Kosmetische Zusammensetzung, **dadurch gekennzeichnet, dass** sie enthält:
- L-2-Oxothiazolidin-4-carbonsäure (I),
- mindestens ein Polyol (II) und
- Wasser,
wobei die Gewichtsanteile der Komponenten (I) und (II) als [I] bzw. [II] bezeichnet werden und die Beziehung [I]/[II] ≤ 1/4 erfüllen.

6. Kosmetische Zusammensetzung nach Anspruch 5, **dadurch gekennzeichnet, dass** der Wasseranteil kleiner oder gleich 35 Gew.-%, vorzugsweise kleiner oder gleich 30 Gew.-% und noch bevorzugter kleiner oder gleich 20 Gew.-%, bezogen auf das Gesamtgewicht der Zusammensetzung, ist.

7. Zusammensetzung nach einem der Ansprüche 5 und 6, **dadurch gekennzeichnet, dass** sie zum Depigmentieren oder zum Bleichen der menschlichen Haut oder der Körperbehaarung und Haare von Personen bestimmt ist.

8. Zusammensetzung nach einem der Ansprüche 5 und 6, **dadurch gekennzeichnet, dass** sie Haarausfall vorbeugen oder Haarwuchs stimulieren soll.

9. Zusammensetzung nach einem der Ansprüche 5 bis 8, **dadurch gekennzeichnet, dass** sie 0,01 bis 10 Gew.-%, vorzugsweise 0,1 bis 5 Gew.-% und noch bevorzugter 0,5 bis 3 Gew.-% L-2-Oxothiazolidin-4-carbonsäure, bezogen auf das Gesamtgewicht der Zusammensetzung, enthält.

10. Zusammensetzung nach einem der Ansprüche 5 bis 9, **dadurch gekennzeichnet, dass** die wässerige Phase durch Einstellen ihres pH-Werts auf einen Wert im Bereich von 6 bis 8 neutralisiert wird.

11. Verwendung mindestens eines Polyols zur Stabilisierung von Procystein.

12. Verfahren zur Stabilisierung von Procystein in einer kosmetischen Zusammensetzung, wobei das Verfahren darin besteht, mindestens eine Verbindung, die unter den Polyolen ausgewählt ist, zu der Zusammensetzung zu geben.

## Claims

1. Combination, **characterized in that** it consists of at least L-2-oxothiazolidine-4-carboxylic acid denoted as compound (I), with at least one compound belonging to the polyol class, denoted as compound (II), the amounts by weight of compounds (I) and (II) being respectively denoted as [I] and [II] and following the relationship [I]/[II] ≤ 1/4.

2. Combination according to Claim 1, **characterized in that** [I]/[II] ≤ 1/5, preferably [I]/[II] ≤ 1/10.

3. Combination according to either of the preceding claims, **characterized in that** the polyol is a compound of linear, branched or cyclic, saturated or unsaturated alkyl type having from 2 to 12 carbon atoms, bearing at least two -OH functions on the alkyl chain, as well as the polymers of these polyhydroxylated alkyl compounds.

4. Combination according to the preceding claim, **characterized in that** the polyol is chosen from: glycerol, ethylene glycol, propylene glycol, polymers and copolymers of glycerol, of ethylene glycol and of propylene glycol, sorbitol, hexylene glycol, butylene glycol, pentylene glycol, butyldiglycol, 1,2,3-trihydroxyhexane.

5. Cosmetic composition, **characterized in that** it comprises:
- L-2-oxothiazolidine-4-carboxylic acid (I)
- at least one polyol (II)
- water
the amounts by weight of the components (I) and (II) being respectively denoted as [I] and [II] and following: [I]/[II] ≤ 1/4.

6. Cosmetic composition according to Claim 5, **characterized in that** the amount of water is less than or equal to 35% by weight relative to the total weight of the composition, preferably less than or equal to 30%, even more preferably less than or equal to 20%.

7. Composition according to either of Claims 5 and 6, **characterized in that** it is intended for the depigmentation or bleaching of human skin or of human head hair or other hair.

8. Composition according to either of Claims 5 and 6, **characterized in that** it is intended to prevent hair loss or to stimulate the regrowth of the hair.

9. Composition according to any one of Claims 5 to 8, **characterized in that** it comprises from 0.01 to 10% by weight of L-2-oxothiazolidine-4-carboxylic acid, preferably from 0.1 to 5%, even more preferably from 0.5 to 3%, by weight relative to the total weight of the composition.

10. Composition according to any one of Claims 5 to 9, **characterized in that** the aqueous phase is neutralized by adjusting its pH to a value between 6 and 8.

11. Use of at least one polyol to stabilize procysteine.

12. Process for stabilizing procysteine in a cosmetic composition, this process consisting in incorporating at least one compound chosen from polyols into the said composition.
